# EUROPEAN PATENT APPLICATION

(11) **EP 4 413 927 A1**
(43) Date of publication of application: **14.08.2024**
(21) Application number: 23305176.2
(22) Date of filing: 09.02.2023
(51) Int. Cl.: A61B 6/00

(54) **PROVIDING GUIDANCE FOR ANGIOGRAPHIC SEQUENCE IMAGING**

(71) Applicant: Koninklijke Philips N.V., 5656 AG Eindhoven (NL)
(72) Inventor: AUVRAY, Vincent, 5656 Eindhoven (NL); LOBANTSEV, Artem, 5656 Eindhoven (NL); ALLAIRE, Stéphane, 5656 Eindhoven (NL); POPOFF, Alexandre, 5656 Eindhoven (NL); OLIVAN BESCOS, Javier, 5656 Eindhoven (NL); COUTEAUX, Vincent, 5656 Eindhoven (NL)
(74) Representative: Philips Intellectual Property & Standards

(57) **Abstract**

The subject-matter of the present disclosure relates to a computer-implemented method of providing guidance for angiographic sequence imaging. The computer-implemented method comprises receiving a plurality of angiogram images of a region of interest during angiographic sequence imaging, wherein each of the angiogram images of the plurality of angiogram images is associated with a viewing perspective of a coronary structure in the region of interest, identifying one or more segments of the coronary structure in each angiogram image of the plurality of angiogram images, and providing an indication that one or more of the one or more identified coronary segments requires one or more additional angiogram images associated with a different viewing perspective.

## Description

### FIELD OF THE INVENTION

The present invention relates to a computer-implemented method of providing guidance for angiographic sequence imaging, a computer-implemented method of providing guidance for percutaneous coronary intervention, a transitory or non-transitory computer-readable storage medium, and a system.

### BACKGROUND

During angiographic sequence imaging of a 3D structure of coronary arteries, medical professionals need to observe each relevant coronary segment from at least two different X-ray views. Without obtaining at least two different X-ray views, it is difficult to correctly identify segments of the coronary artery structures not discernible from every 2D projections, such as stenoses. This is because some stenoses are highly isotropic, and thus appear in 2D clearly from some perspective, but are virtually invisible from others. As a result, a lesion may be visible from one orientation of the segment but not visible from another orientation of the segment. In such cases, a single image may provide false negative diagnoses of certain stenoses. As a result, it is difficult to disambiguate coronary segments requiring interventional endo-vascular treatment, such as percutaneous coronary intervention.

It is an aim of the present invention to improve on the prior art.

### SUMMARY

According to a first aspect of the present invention, there is provided a computer-implemented method of providing guidance for angiographic sequence imaging, the computer-implemented method comprising: receiving a plurality of angiogram images of a region of interest during angiographic sequence imaging, wherein each of the angiogram images of the plurality of angiogram images is associated with a viewing perspective of a coronary structure in the region of interest; identifying one or more segments of the coronary structure in each angiogram image of the plurality of angiogram images; and providing an indication that one or more of the one or more identified coronary segments requires one or more additional angiogram images associated with a different viewing perspective.

Providing the indication provides guidance to the clinician for capturing additional angiogram images of different viewing perspectives so that stenoses can be identified more accurately, in comparison to a case where there is an insufficient number of angiogram images from different viewing perspectives.

In an embodiment, the viewing perspective comprises one or more rotational angles of an X-ray detector at the time of capturing the respective angiogram image of the plurality of angiogram images, or an angiogram view classification.

The X-ray detector may be an X-ray detector from a C-arm device.

In an embodiment, the computer-implemented method may further comprise: determining the viewing perspective associated with each of the angiogram images by applying each angiogram image to a neural network model trained to determine the viewing perspective of an angiogram image; or when the viewing perspective comprises one or more rotational angles of an X-ray detector at the time of capturing the respective angiogram image of the plurality of angiograph images, determining an angiogram view classification associated with each of the angiogram images using metadata associated with each of the angiogram images, wherein the metadata describes the one or more angles of the X-ray detector.

In either case, a workload of the clinician is reduced. In the case of using a neural network, the viewing perspective is determined even if metadata is unavailable. The metadata, where available, means that there is no need to train the neural network and computation is more efficient.

In an embodiment, the identifying one or more segments of the coronary structure in each angiogram image comprises: retrieving a coronary segment associated with the viewing perspective from a database mapping a viewing perspective to a coronary segment.

Retrieving the coronary segment from the database is a computationally efficient way to obtain the coronary segment.

In an embodiment, the identifying one or more segments of the coronary structure in each angiogram image comprises: applying each angiogram image to a neural network model trained to identify one or more coronary segments from an angiogram image. A well-trained neural network omits the need for a reliable identification of the viewing angle.

In an embodiment, the computer-implemented method may further comprise: determining, for each of the one or more segments, a number of different viewing perspectives associated with each of the identified one or more segments of the coronary structure in the plurality of angiogram images; and comparing the determined number of different viewing perspectives associated with each of the one or more coronary segments to a threshold number of viewing perspectives, wherein the providing the indication that the one or more coronary segments that require one or more additional angiogram images associated with a different viewing perspective comprises identifying the one or more coronary segments where the number of different viewing perspectives is below the threshold number.

In an embodiment, the identifying one or more segments of the coronary structure in each angiogram image of the plurality of angiogram images comprises identifying a coronary tree from each angiogram image using a neural network. Identifying the coronary tree may mean identifying a left coronary tree or a right coronary tree.

By identifying the coronary tree, it is easier to then identify the coronary segment, since left and right hand trees have different arrangements of segments. It is mostly beneficial in the case where the segment visibility is defined by the acquisition angles (as opposed to assessed by a network).

If you know the angles and use guidelines to determine which segments are well visible, the one information you need from the image is whether it is the right or the left tree that is imaged.

In an embodiment, the providing the indication comprises displaying a map of the coronary structure, wherein the map comprises the one or more identified coronary segments requiring one or more additional angiogram images associated with a different viewing perspective differently to the remaining one or more coronary segments.

In an embodiment, the displaying the one or more identified coronary segments requiring one or more additional angiogram images associated with a different viewing perspective differently to the remaining one or more coronary segments comprises displaying the one or more identified coronary segments requiring the additional angiogram image associated with a different viewing perspective in a different color to the other coronary segments. Color coding the segments in the map is intuitive for a clinician to interpret quickly and easily.

In an embodiment, the map of the coronary structure comprises at least one of a pictorial map and a textual map.

According to an aspect, there is provided a computer-implemented method of providing guidance for percutaneous coronary intervention, the computer-implemented method comprising: guiding angiographic sequence imaging according to the computer-implemented method of any preceding aspect or embodiment; receiving an image of the region of interest during a percutaneous coronary intervention; displaying, on a display, the image of the region of interest together with the indication of the one or more identified coronary segments that require one or more angiogram images associated with a different viewing perspective.

In this way, a clinician performing the intervention can understand in real-time if a segment is a suitable candidate for balloon placement, for example.

In an embodiment, the computer-implemented method further comprises: determining a coronary segment location of an intervening tool during the percutaneous coronary intervention in the received image, wherein the providing the indication of one or more identified coronary segments that require one or more additional angiogram images associated with a different viewing perspective comprises: responsive to determining that the intervening tool is located in one of the one or more identified coronary segments that require one or more additional angiogram images associated with a different viewing perspective, providing an indication that the identified coronary segment where the intervening tool is located requires one or more additional angiogram images associated with a different viewing perspective.

In an embodiment, the computer-implemented method further comprises: aligning the image of the region of interest received during the percutaneous coronary intervention with one of the plurality of angiogram images of the region of interest received during angiographic sequence imaging; wherein the determining, based on the image of the region of interest received during the percutaneous coronary intervention, the coronary segment location of an intervening tool during the percutaneous coronary intervention is based on the aligning the image of the region of interest received during the percutaneous coronary intervention with one of the plurality of angiogram images of the region of interest received during angiographic sequence imaging.

According to an aspect, there is provided a transitory or non-transitory computer-readable storage medium storing instructions which, when executed by a processor, cause the processor to perform the computer-implemented method of any preceding claim.

According to an aspect, there is provided a system comprising: storage including the non-transitory computer-readable storage medium of the preceding aspect stored thereon; at least one processor configured to execute the instructions from the non-transitory computer-readable storage medium; and a display configured to: display the indication of one or more identified coronary segments that require one or more additional angiogram images associated with a different viewing perspective, and/or display the received image of the region of interest together with the indication of the identified coronary segments that requires one or more additional angiogram images associated with a different viewing perspective.

These and other aspects of the present invention will be apparent from and elucidated with reference to the embodiment(s) described hereinafter.

### BRIEF DESCRIPTION OF DRAWINGS

The embodiments of the present inventions may be best understood with reference to the accompanying Figs, in which:
Fig. 1 shows a schematic cross-sectional diagram of a C-arm device;
Fig. 2 shows a block diagram of a system for performing a method according to an embodiment of the invention;
Fig. 3 shows flow chart of steps involved in a computer-implemented method of providing guidance for angiographic sequence imaging and a computer-implemented method of providing guidance for percutaneous coronary intervention according to embodiments of the invention;
Fig. 3A shows the display 310 from Fig. 3 in more detail;
Fig. 4 shows a schematic diagram illustrating respective well visible and foreshortened coronary segments from angiogram images captured during angiographic sequence imaging from Fig. 3;
Fig. 5 shows a schematic diagram illustrating left and right coronary trees;
Fig. 6 shows a flow chart summarizing the computer-implemented method of providing guidance for angiographic sequence imaging from Fig. 3; and
Fig. 7 shows a flow chart summarizing the computer-implemented method of providing guidance for percutaneous coronary intervention from Fig. 3.

### DESCRIPTION OF EMBODIMENTS

At least some of the example embodiments described herein may be constructed, partially or wholly, using dedicated special-purpose hardware. Terms such as 'component', 'module' or 'unit' used herein may include, but are not limited to, a hardware device, such as circuitry in the form of discrete or integrated components, a Field Programmable Gate Array (FPGA) or Application Specific Integrated Circuit (ASIC), which performs certain tasks or provides the associated functionality. In some embodiments, the described elements may be configured to reside on a tangible, persistent, addressable storage medium and may be configured to execute on one or more processors. These functional elements may in some embodiments include, by way of example, components, such as software components, object-oriented software components, class components and task components, processes, functions, attributes, procedures, subroutines, segments of program code, drivers, firmware, microcode, circuitry, data, databases, data structures, tables, arrays, and variables. Although the example embodiments have been described with reference to the components, modules and units discussed herein, such functional elements may be combined into fewer elements or separated into additional elements. Various combinations of optional features have been described herein, and it will be appreciated that described features may be combined in any suitable combination. In particular, the features of any one example embodiment may be combined with features of any other embodiment, as appropriate, except where such combinations are mutually exclusive. Throughout this specification, the term "comprising" or "comprises" means including the component(s) specified but not to the exclusion of the presence of others.

The methods described herein may be computer-implemented methods. In particular, the methods may include at least a computer-implemented method of generating a composite video and a computer-implemented method of training one or more machine learning algorithms. The computer which implements the methods may include a storage, e.g. memory, and a processor. The computer may be a hardware computer and thus the storage and the processor may respectively be a hardware storage and a hardware processor.

The computer-implemented methods may be provided as instructions stored on a transitory, or non-transitory, computer-readable medium. The computer-readable medium may be stored in the storage of the computer. When the instructions are executed by the processor, the instructions cause the processor to perform any of the method steps descried herein.

Angiographic imaging is a diagnostic medical imaging technique that utilizes X-rays to observe coronary structures, such as coronary trees or arteries. An angiogram image is a 2D X-ray image of coronary structures comprising a plurality of coronary segments. During angiographic imaging, the medical professional aims to image the coronary structures from a number of viewing perspectives in order to obtain angiograms with a view each of the coronary segments of the coronary structures. If only one viewing perspective of a coronary structure is obtained, information present in the dimension orthogonal to the imaging plane is not obtained. For example, a dangerous narrowing of a coronary segment (i.e., a stenosis) would not be observable in a single viewing perspective of a coronary structure if it spreads orthogonally to the imaging plane.

Once identified, stenoses are treatable using endo-vascular procedures. For example, percutaneous coronary intervention treatment involves the insertion, by guidewire, of a stent and/or balloon into the patient's coronary structure to widen and support the narrowed coronary segment. Contrast injection is not typically used by a medical professional during percutaneous coronary intervention. Instead, alternative imaging techniques are used to obtain images in which the interventional tools are visible.

To enable angiographic sequence imaging, a patient is first injected with a contrast agent that is opaque to X-rays. A plurality of angiographic images is then obtained using an X-ray detecting medical device, such as a C-arm device.

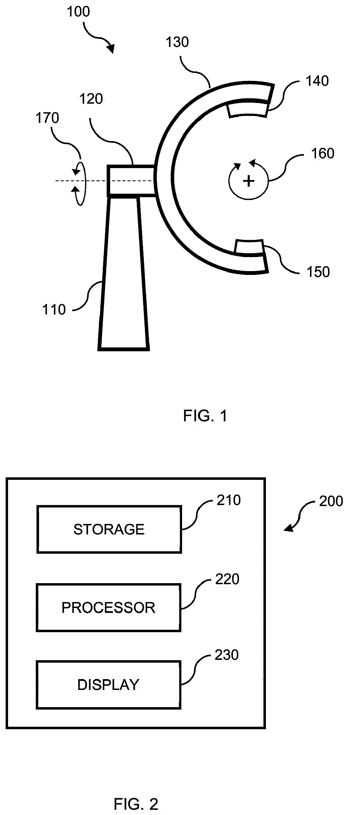

With reference to Fig. 1, a C-arm device 100 typically includes an X-ray source 140 and an X-ray detector 150 arranged at either end of a circular arc shaped arm 130. The source 140 and detector 150 are arranged to oppose one another, thereby forming a line-of-sight between the source 140 and detector 150 such that signals emitted by the source 140 are detectable by the detector 150. During imaging, a patient is positioned on a table surface between the source 140 and detector 150 of the C-arm 130 such that the line of sight impinges the patient's body. For a given position of the line-of-sight relative to the patient's body, the C-arm 130 obtains an image having a particular viewing perspective of the patient's body.

The C-arm 130 is connected to a base structure 110, such as a stand, by means of a supporting arm 120. The C-arm 130 is moveable by at least rotational motion relative to the base structure 110. The C-arm 130 is moveable by translational and rotational motion relative to the table surface and patient thereon. In terms of translational motion, the C-arm is moveable 130 in the horizontal and vertical planes. In terms of rotational motion, the C-arm 130 is moveable by orbital rotation 160 about the geometric center of the circular arc of the C-arm, i.e., rotation within the plane of the C-shaped arm. Additionally, the C-arm is moveable by angular rotation 170 about the axis of the supporting arm to provide a propeller-like rotation of the C-arm.

The C-arm 130 is thus repositionable to obtain a plurality of angiograms of a coronary structure, wherein each angiogram is associated with a different viewing perspective relative to the patient. For example, for a particular region of interest, such as a particular translational position of the patient's body, the C-arm 130 can be repositioned by orbital and/or angular rotation. Each angiogram image is thus associated with a viewing perspective comprising one or more of a translational position, an orbital rotation (i.e., orbital angle) 160 and an angulation rotation (i.e., angulation angle) 170 of the C-arm 130. The translational component may be used to position the heart at the C-arm isocenter. Therefore, the C-arm angles are more important regarding identifying which segments are visible.

Metadata for each angiogram image includes the aforementioned viewing perspective information. Angiograms and the corresponding metadata may be stored at the C-arm device 100 and/or at a remote server communicatively linked to the C-arm device 100.

Coronary segments have different levels of visibility at different viewing perspectives. An angiogram view classification corresponds to an orbital and/or angulation angles that provide good visibility of particular coronary structures and segments. Good visibility refers to a viewing perspective associated with minimal cluttering and/or foreshortening of the imaged coronary segments. For example, cluttering refers to the obstruction of the coronary segments due to the presence of different structures within the field of view of the detector (in that case, other vessels). Foreshortening refers to the reduction in length of the coronary segments that occurs when the segment extends at an angle to the imaging plane.

Typically, angiogram view classifications are classified as either a right anterior oblique (RAO) view, a left anterior oblique (LAO) view, or an antero-posterior (AP) view. These view classifications refer to the angulation direction of the C-arm 130 relative to the patient. For example, the RAO view corresponds to an angulation of the C-arm such that the right-hand side of the patient is within the field of view of the detector. Conversely, the LAO view corresponds to an angulation of the C-arm 130 such that the left-hand side of the patient is within the field of view of the detector. The AP view corresponds to an angulation of the C-arm 130 where the detector is vertical to the patient.

Each of the RAO, LAO, and AP views can be further classified as cranial or caudal. A cranial view classification corresponds to an orbital rotation of the C-arm 130 towards the patient's head. Conversely, a caudal view classification corresponds to orbital rotation of the C-arm 130 towards the patient's feet.

The methods disclosed herein may be performed by a system 200 as shown in Fig. 2. The system 200 comprises storage 210 including a non-transitory computer-readable storage medium, a processor 220, and a display 230.

With further reference to Fig. 2, the system is configured to receive a plurality of angiogram images. For example, the system 200 may be communicatively linked to a server that is communicatively linked to the C-arm device 100. The system 200 may receive the angiograms from the server via a wireless connection, such as Bluetooth or wi-fi, or by physical connection, such as cable. Alternatively, the system may receive the angiograms from a storage means directly connected to the system.

With further reference to Fig. 2, the system display 230 is configured to display the indication of one or more identified coronary segments that require one or more additional angiogram images associated with a different viewing perspective and/or display the received image of the region of interest together with the indication of the identified coronary segments that requires one or more additional angiogram images associated with a different viewing perspective. The system display 230 may further be configured to display one or more angiogram images together with the received image of the region of interest and the indication.

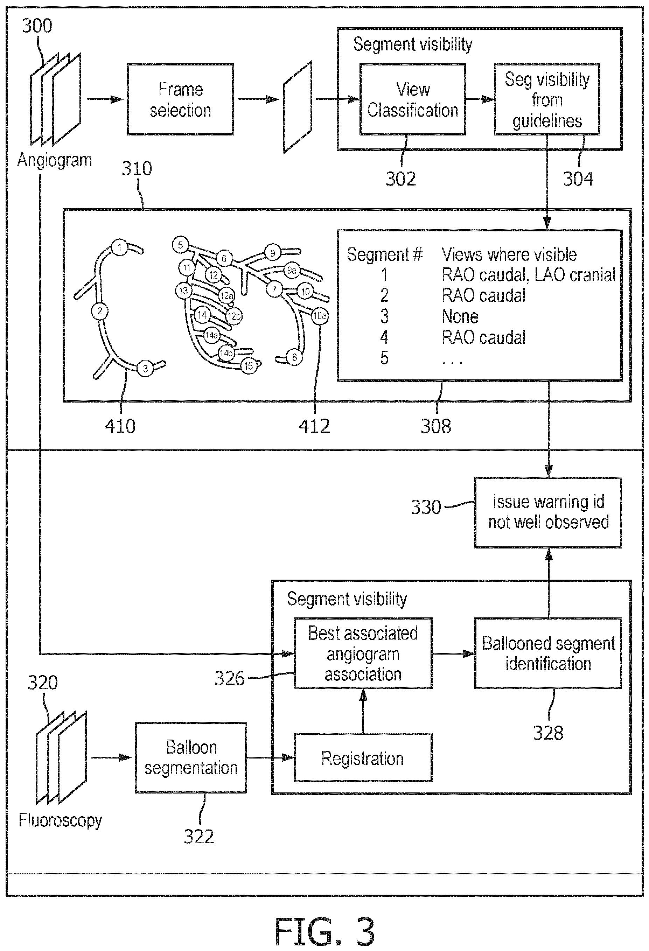

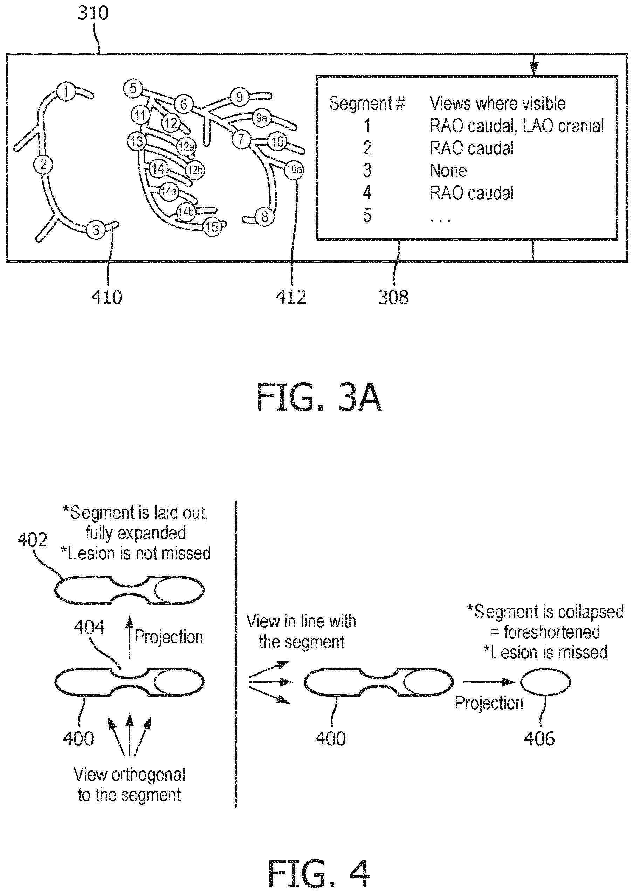

Fig. 3 shows an overview illustrating two computer-implemented methods. A first computer-implemented method is a method of providing guidance for angiographic sequence imaging. A second computer-implemented method is a method of providing guidance for percutaneous coronary intervention.

With reference to Fig. 3, the method of providing guidance during a diagnostic procedure, a plurality of angiogram images 300 are captured using the C-arm during angiographic image sequencing. A viewing perspective 302 of a coronary structure in a region of interest of the angiogram image is determined.

As alluded to above, wherein the viewing perspective comprises one or more rotational angles of an X-ray detector at the time of capturing the respective angiogram image of the plurality of angiogram images, or an angiogram view classification.

When determining the viewing perspective 302, the method may comprise determining the viewing perspective 302 associated with each of the angiogram images 300 by applying each angiogram image to a neural network model trained to determine the viewing perspective of an angiogram image. In other embodiments, when the viewing perspective comprises one or more rotational angles of an X-ray detector at the time of capturing the respective angiogram image of the plurality of angiogram images, the method may comprise determining an angiogram view classification associated with each of the angiogram images 300 using metadata associated with each of the angiogram images. The metadata describes the one or more angles of the X-ray detector at the time when the associated angiogram image was captured.

In other embodiments, the view classification can be determined by applying the angiogram image to a neural network trained to determine a view classification from features in the angiogram image. This neural network may be a first neural network.

The method also comprises identifying one or more segments 304 (or visible segments) of the coronary structure in each angiogram image of the plurality of angiogram images 300.

In some embodiments, this step comprises retrieving a coronary structure associated with the viewing perspective from a database mapping a coronary structure to a viewing perspective. The mapping may be based on medical guidelines. It is possible to apply each angiogram image to a neural network classifier trained to identify a coronary structure, e.g. left or right, from an angiogram image. Using the coronary structure, a more accurate retrieval of the specific one or more segments may be made. The neural network classifier may be a third neural network. The neural network classifier may be a convolutional neural network.

In other embodiments, this step comprises applying each angiogram image to a neural network model trained to identify one or more coronary segments directly from an angiogram image. In this way, there will be no need to retrieve the segments from the database. The neural network may be a second neural network, which may be a convolutional neural network. The neural network may be trained using angiogram images labelled with any coronary segments that are visible in them.

By "visible", we mean that the segment is shown in a manner that does not obscure the segment. For example, the segment is not shown with any foreshortening.

With brief reference to Fig. 4, a segment 400 is shown. An orthogonal projection 402 of the segment as shown and a lesion 404 can be seen on the projection. Such an orthogonal projection 402 is considered to be satisfactory since there is no foreshortening of the segment 400. In other words, in the orthogonal projection, the segment is well visible.

A longitudinal projection 406 is also shown in Fig. 4, where the segment is shown in-line in the angiogram. In other words, the detector is oriented in line with a longitudinal axis of the segment. The longitudinal projection 406 shows a foreshortened segment where the lesions are not visible. In other words, where there is foreshortening, the segment is not well visible.

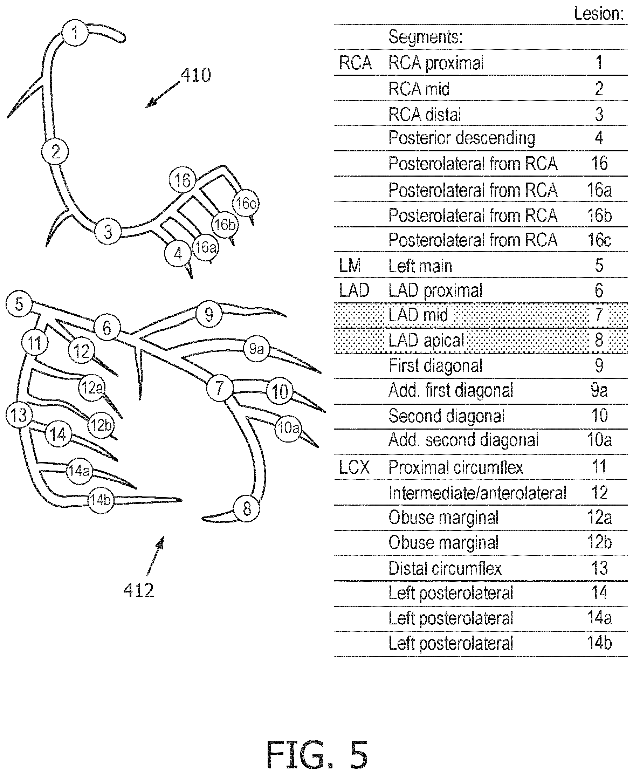

This may be best understood with reference to Fig. 5, which shows a right coronary structure, or tree, 410 and a left coronary structure, or tree, 412. Each tree 410, 412, includes multiple segments. The segments between trees are different. The right tree 410 includes segments 1 to 4 and 16 to 16c. The left tree 412 includes segments labelled 5 to 14b. As shown in Fig. 5, because the trees extend three-dimensionally, some orientations of the detector will mean that the segment is anywhere from orthogonal to longitudinal to a longitudinal axis of the segment.

With further reference to Fig. 3, the method may also comprise determining, for each of the one or more segments, a number of different viewing perspectives associated with each of the identified one or more segments of the coronary structure in the plurality of angiogram images; and comparing the determined number of different viewing perspectives associated with each of the one or more coronary segments to a threshold number of viewing perspectives. In some embodiments, the threshold number of viewing perspectives may be two. In other words, the method may check to see if there are two different viewing perspectives for each segment. The clinical recommendation is to ensure that each segment has been observed from two different viewing perspective at least, to ensure that no significant stenosis characteristic risk to be missed. The different viewing perspectives may be substantially different. For example, the different viewing perspectives may be viewing perspectives that show the segment substantially orthogonally to one another.

The method may comprise providing an indication that one or more of the one or more identified coronary segments requires one or more additional angiogram images associated with a different viewing perspective. This may be achieved by displaying a map on a display 310. The display 310 may correspond to the display 230 from Fig. 2. Fig. 3A shows an enlarged view of the display 310 from Fig. 3.

The map may include a pictorial map showing the coronary structure (left and right coronary trees, 412, 410) on a display together with a textual map 308 of the coronary structure. The textual map 308 may be a table. The table may include two columns. A first column includes a segment number, e.g. the segment numbers from Fig. 5. The second column may include a description, or name, or label, of a viewing perspective of an angiogram that has been captured and the segment is visible in it. The textual map 308 may be color coded. In other embodiments, the pictorial map may also be color coded. The color coding may indicate a number of views for that segment. The color coding may be achieved by color coding the segment number in the first column and the label of the viewing perspective in the second column. The color coding may include red where there are no angiogram images captured showing the segment, yellow, orange, or amber, where there is one angiogram image showing the segment (or where the number of angiogram images showing the segment is less than the threshold), and green where the number of angiogram images showing the segment at different orientations is equal to or greater than the threshold.

In this way, the map comprises the one or more identified coronary segments requiring one or more additional angiogram images associated with a different viewing perspective differently to the remaining one or more coronary segments, and wherein the providing the indication that the one or more coronary segments that require one or more additional angiogram images associated with a different viewing perspective comprises identifying the one or more coronary segments where the number of different viewing perspectives is below the threshold number.

Also, as alluded to above, the displaying the one or more identified coronary segments requiring one or more additional angiogram images associated with a different viewing perspective differently to the remaining one or more coronary segments comprises displaying the one or more identified coronary segments requiring the additional angiogram image associated with a different viewing perspective in a different color to the other coronary segments

In other embodiments, the providing the indication may be active in the sense of an active warning for the clinician to capture different views of any segments where the number of different views is below the threshold number.

In the second method, a plurality of fluoroscopy images 320 are captured during a percutaneous coronary intervention procedure.

The method may comprise determining a coronary segment location of an intervening tool during the percutaneous coronary intervention in the received image. The intervening tool may be a deflated balloon. This may first involve balloon segmentation 322, which may be performed using an object identification model. The object identification model may be a "you only look once" (YOLO) algorithm, or a Retinanet algorithm, for example.

The fluoroscopy image does not show the vessel because contrast media is not used during the procedure, unlike the diagnostic procedure where a contrast agent is used. To determine the segment location of the balloon, the fluoroscopic image can be overlayed onto an angiogram having the same viewing perspective. This overlay is achieved using an association algorithm 326. Next, the coronary segment location of the balloon is determined using one or more of the same method described above for the diagnostic method. In other words, a segment location method 328 is used to determine the segment location of the interventional tool, e.g. balloon.

In this way, the determining the coronary segment location of an intervening tool during the percutaneous coronary intervention is based on aligning the image of the region of interest received during the percutaneous coronary intervention with one of the plurality of angiogram images of the region of interest received during angiographic sequence imaging.

There may be a display 330 during the intervention procedure. The display may be the same display as display 310 in some embodiments and/or may be the same display as display 230 from

Fig. 2 in some embodiments. The display 330 may display the fluoroscopic image and the map (from the diagnostic procedure) side by side. In this way, the method may comprise displaying, on the display 330, the image of the region of interest (fluoroscopic image)_together with the indication of the one or more identified coronary segments that require one or more angiogram images associated with a different viewing perspective. This approach to displaying the indication is passive in the sense that the clinician is able to make up their own mind about suitability of a segment for deploying the balloon.

In other embodiments, the indication may be displayed actively. In other words, responsive to determining that the intervening tool is located in one of the one or more identified coronary segments that require one or more additional angiogram images associated with a different viewing perspective, the method may comprise providing an indication that the identified coronary segment where the intervening tool is located requires one or more additional angiogram images associated with a different viewing perspective. This may be achieved by outputting a warning when the balloon has reached a steady state position in a segment where there are too few angiogram images of different viewing perspectives captured during the diagnostic phase.

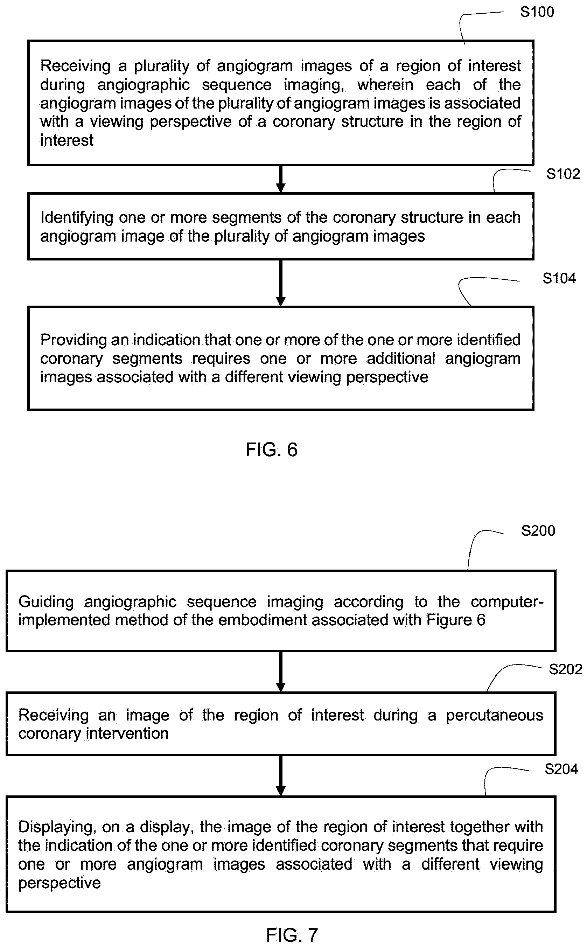

The foregoing methods may be summarized with reference to Figs 6 and 7.

With reference to Fig. 6, there is provided a computer-implemented method of providing guidance for angiographic sequence imaging, according to one or more embodiments. The computer-implemented method comprises: receiving S 100 a plurality of angiogram images of a region of interest during angiographic sequence imaging, wherein each of the angiogram images of the plurality of angiogram images is associated with a viewing perspective of a coronary structure in the region of interest; identifying S102 one or more segments of the coronary structure in each angiogram image of the plurality of angiogram images; and providing S 104 an indication that one or more of the one or more identified coronary segments requires one or more additional angiogram images associated with a different viewing perspective

With reference to Fig. 7, there is provided a computer-implemented method of providing guidance for percutaneous coronary intervention. The computer-implemented method comprises: guiding S200 angiographic sequence imaging according to the computer-implemented method of the embodiment associated with Fig. 6; receiving S202 an image of the region of interest during a percutaneous coronary intervention; and displaying S204, on a display, the image of the region of interest together with the indication of the one or more identified coronary segments that require one or more angiogram images associated with a different viewing perspective

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measured cannot be used to advantage. Any reference signs in the claims should not be construed as limiting the scope.

## Claims

1. A computer-implemented method of providing guidance for angiographic sequence imaging, the computer-implemented method comprising:
receiving (S100) a plurality of angiogram images (300) of a region of interest during angiographic sequence imaging, wherein each of the angiogram images of the plurality of angiogram images is associated with a viewing perspective of a coronary structure in the region of interest;
identifying (S102) one or more segments of the coronary structure in each angiogram image of the plurality of angiogram images; and
providing (S104) an indication that one or more of the one or more identified coronary segments requires one or more additional angiogram images associated with a different viewing perspective.

2. The computer-implemented method of claim 1, wherein the viewing perspective comprises one or more rotational angles of an X-ray detector (150) at the time of capturing the respective angiogram image of the plurality of angiogram images (300), or an angiogram view classification.

3. The computer-implemented method of any preceding claim, further comprising:
determining the viewing perspective associated with each of the angiogram images by applying each angiogram image to a neural network model trained to determine the viewing perspective of an angiogram image; or
when the viewing perspective comprises one or more rotational angles of an X-ray detector at the time of capturing the respective angiogram image of the plurality of angiograph images, determining an angiogram view classification associated with each of the angiogram images using metadata associated with each of the angiogram images, wherein the metadata describes the one or more angles of the X-ray detector.

4. The computer-implemented method of any preceding claim, wherein the identifying one or more segments of the coronary structure in each angiogram image comprises:
retrieving a coronary segment associated with the viewing perspective from a database mapping a coronary segment to a viewing perspective.

5. The computer-implemented method of any of claims 1 to 3, wherein the identifying one or more segments of the coronary structure in each angiogram image comprises:
applying each angiogram image to a neural network model trained to identify one or more coronary segments from an angiogram image.

6. The computer-implemented method of any preceding claim, further comprising:
determining, for each of the one or more segments, a number of different viewing perspectives associated with each of the identified one or more segments of the coronary structure in the plurality of angiogram images; and
comparing the determined number of different viewing perspectives associated with each of the one or more coronary segments to a threshold number of viewing perspectives,
wherein the providing the indication that the one or more coronary segments that require one or more additional angiogram images associated with a different viewing perspective comprises identifying the one or more coronary segments where the number of different viewing perspectives is below the threshold number.

7. The computer-implemented method of any preceding claim, wherein the identifying one or more segments of the coronary structure in each angiogram image of the plurality of angiogram images comprises identifying a coronary tree (410, 412) from each angiogram image using a neural network.

8. The computer-implemented method of any preceding claim, wherein the providing the indication comprises displaying a map of the coronary structure, wherein the map comprises the one or more identified coronary segments requiring one or more additional angiogram images associated with a different viewing perspective differently to the remaining one or more coronary segments.

9. The computer-implemented method of claim 8, wherein the displaying the one or more identified coronary segments requiring one or more additional angiogram images associated with a different viewing perspective differently to the remaining one or more coronary segments comprises displaying the one or more identified coronary segments requiring the additional angiogram image associated with a different viewing perspective in a different color to the other coronary segments.

10. The computer-implemented method of any of claims 7 to 9, wherein the map of the coronary structure comprises at least one of a pictorial map and a textual map (308).

11. A computer-implemented method of providing guidance for percutaneous coronary intervention, the computer-implemented method comprising:
guiding (S200) angiographic sequence imaging according to the computer-implemented method of any preceding claim;
receiving (S202) an image of the region of interest during a percutaneous coronary intervention;
displaying (S204), on a display (230), the image of the region of interest together with the indication of the one or more identified coronary segments that require one or more angiogram images associated with a different viewing perspective.

12. The computer-implemented method of claim 11, further comprising:
determining a coronary segment location of an intervening tool during the percutaneous coronary intervention in the received image,
wherein the providing the indication of one or more identified coronary segments that require one or more additional angiogram images associated with a different viewing perspective comprises:
responsive to determining that the intervening tool is located in one of the one or more identified coronary segments that require one or more additional angiogram images associated with a different viewing perspective, providing an indication that the identified coronary segment where the intervening tool is located requires one or more additional angiogram images associated with a different viewing perspective.

13. The computer-implemented method of claim 12, further comprising:
aligning the image of the region of interest received during the percutaneous coronary intervention with one of the plurality of angiogram images of the region of interest received during angiographic sequence imaging;
wherein the determining, based on the image of the region of interest received during the percutaneous coronary intervention, the coronary segment location of an intervening tool during the percutaneous coronary intervention is based on the aligning the image of the region of interest received during the percutaneous coronary intervention with one of the plurality of angiogram images of the region of interest received during angiographic sequence imaging.

14. A transitory or non-transitory computer-readable storage medium storing instructions which, when executed by a processor, cause the processor to perform the computer-implemented method of any preceding claim.

15. A system comprising:
storage (210) including the non-transitory computer-readable storage medium of claim 14 stored thereon;
at least one processor (220) configured to execute the instructions from the non-transitory computer-readable storage medium; and
a display (230) configured to:
display the indication of one or more identified coronary segments that require one or more additional angiogram images associated with a different viewing perspective, and/or
display the received image of the region of interest together with the indication of the identified coronary segments that requires one or more additional angiogram images associated with a different viewing perspective.
